# EUROPEAN PATENT SPECIFICATION

(11) **EP 4 203 933 B1**
(45) Date of publication and mention of the grant of the patent: **25.11.2026**
(21) Application number: 21862868.3
(22) Date of filing: 27.08.2021
(51) Int. Cl.: C07C 51/235, A61K 31/191, A61K 31/20, A61K 33/06

(54) **METHODS FOR THE PRODUCTION OF L-THREONIC ACID SALTS FROM L-XYLONIC ACID**
VERFAHREN ZUR HERSTELLUNG VON L-THREONSÄURESALZEN AUS L-XYLONSÄURE
PROCÉDÉS DE PRODUCTION DE SELS D'ACIDE L-THRÉONIQUE À PARTIR D'ACIDE L-XYLONIQUE

(30) Priority: 28.08.2020 US 202062706632 P
(43) Date of publication of application: 05.07.2023
(73) Proprietor: DFI USA, LLC, Greenacres, WA 99016 (US)
(72) Inventor: STAPLEY, Jonathan, Mercer Island, Washington 98040 (US); GENDERS, David, Lancaster, New York 14086 (US)
(74) Representative: Fuchs Patentanwälte Partnerschaft mbB
(86) International application number: PCT/US2021/048057
(87) International publication number: WO 2022/047231

(56) References cited:
- CN-A- 1 267 662
- CN-A- 106 083 567
- US-A1- 2004 254 368
- US-A1- 2019 328 689
- US-B2- 6 894 199
- US-B2- 7 955 489
- US-B2- 9 702 047

## Description

### TECHNICAL FIELD

The present disclosure relates to a process of producing high purity L-threonic acid salts from L-xylonic acid, comprising providing L-xylonic acid, decarboxylating L-xylonic acid to produce L-threose, and oxidizing the L-threose to produce L-threonic acid salts. The present disclosure also relates to the recycle of unreacted materials within the process.

### BACKGROUND

L-Threonic acid is a valuable commercial sugar acid, salts of which are employed in pharmaceutical and supplement preparations. Magnesium and calcium L-threonates in particular have been an important product in the supplement industry. Salts of L-threonic acid are traditionally produced from ascorbic acid by alkaline degradation or peroxide decarboxylation as in US Patents Nos. 4,822,816, 4,968,716, and Chinese Patent Nos. 1,267,662A, and 106,083,567A. As these methods use costly reagents, have poor selectivity, and are carried out in dilute solutions, there remains a need to produce high purity L-Threonic acid salts in a selective manner from readily available raw materials.

### SUMMARY

A method has now been found for the preparation of L-Threonic Acid Salts, this being the object of the present invention, which is characterized in that it comprises the following stages: L-xylonic acid is provided and decarboxylated to yield L-threose; the L-threose is oxidized to L-threonic acid, preferably using a noble metal catalyst, and more preferably after the removal of unreacted acid. Preferably the method comprises the following recycle steps to minimize waste and reduce operating cost: the separation of unreacted L-xylonic acid from the L-threose, and reuse as a raw material for L-threose production, preferably eluting the reaction products through a separation zone containing cation exchange resin.

### DETAILED DESCRIPTION

### Definitions

As used herein, the term L-xylonic acid refers to the aldonic acid L-xylonic acid, and salts thereof. A salt of L-xylonic acid is referred to as a L-xylonate. For example the sodium salt of L-xylonic acid is sodium L-xylonate.

As used herein, the term L-threose refers to L-threose. L-threose is a rare sugar and is currently not produced industrially.

As used herein, the term KLG refers to the 2-keto-L-gulonic acid, and salts thereof. A salt of 2-keto-Lgulonic acid is referred to as a 2-keto-L-gulonate. For example the sodium salt of 2-keto-L-gulonic acid is sodium 2-keto-L-gulonate. KLG is an intermediate in ascorbic acid production.

As used herein, the term L-sorbose refers to the ketose L-sorbose. L-sorbose is an intermediate in ascorbic acid production.

As used herein, the term metal hydroxide refers to a hydroxide salt of sodium, potassium, lithium, magnesium, or calcium.

The term "decarboxylation" as used herein refers to the removal of a carboxyl group (-COOH) by a chemical reaction or physical process. Typical products of a decarboxylation reaction may include carbon dioxide (CO2) or formic acid.

The term "electrochemical" refers to chemical reactions that take place at the interface of an electrical conductor (an electrode) and an ionic conductor (the electrolyte). Electrochemical reactions can create a voltage potential between two conducting materials (or two portions of a single conducting material), or can be caused by application of external voltage. In general, electrochemistry deals with situations where an oxidation and a reduction reaction is separated in space.

The term "electrolytic" as used herein refers to an electrochemical oxidation or reduction reaction that results in the breaking of one or more chemical bonds. Electrolytic reactions as used herein preferably describe reactions occurring as a product of interaction with a cathode or anode.

### Providing L-Xylonic Acid

The present method requires providing L-xylonic acid as a raw material for the production of L-threonic acid. L-xylonic acid can be produced through different routes, for instance by oxidation of L-sorbose as in Isbell et al., J. Res. Nat. Bur. Stand. 29 (1942) 227-232 and US Patent No. 6,894,199 B2. KLG can be decarboxylated to yield xylonic acid in good yields using alkaline hydrogen peroxide as in Isbell et al. Carbohydr. Res. 36 (1974) 283-291, and US Patent No. 6,894,199 B2. L-xylose, like all aldoses, can be oxidized to its corresponding aldonic acid, L-xylonic acid, via noble metal catalysis. KLG can also be decarboxylated electrochemically to produce L-xylonic acid.

### Xylonic Acid Decarboxylation

Aldonic acids can be decarboxylated using various chemical systems including hypochlorous acid, and hydrogen peroxide systems, as in US Patent No. 5,714,602. Aldonic acids can be selectively decarboxylated to yield sugars having one less carbon, using electrochemical cells as in US Patents Nos., 7,598,374, 7,955,489, and 9,702,047. For example, U.S. Patent No. 7,955,489, describes the electrolytic decarboxylation of aqueous D- or L-arabinonic to yield erythrose. The electrochemical decarboxylation of sugar acids is a two-electron oxidation.

In one embodiment of the current disclosure, the step of xylonic acid decarboxylation is performed using an electrochemical cell. Preferably the electrochemical cell comprises and anode, cathode, and cation exchange membrane which separates the anode chamber and the cathode chamber. In one embodiment the L-xylonic acid is provided in the cell by the concurrent electrochemical decarboxylation of KLG.

### L-Threose Oxidation

Aldoses can be selectively oxidized to aldonate salts by chemical oxidation. Nobel metal catalysts have been widely described in the literature, and gold catalysts in particular have shown to be very effective for this oxidation as described by Theilecke, et al, Catalysis Today, 115-120 (2007). L-Threose has been oxidized to the magnesium L-threonate as described in PCT Application No. PCT/US2020/070085.

In one embodiment of the current disclosure, the step of L-threose oxidation is performed using pressurized oxygen or air in the presence of a metal hydroxide. Preferably the oxidation is performed using a gold-based catalyst. More preferably more than 95% of the L-threose is converted to L-threonic acid.

### Threose Purification

Mixtures of sugars, sugar acids and polyols are often separated chromatographically, and industrially using simulated moving bed chromatography. A separation zone containing cation exchange resin can be used as the chromatographic medium, with various cation species result in different sugar elution rates. Calcium has widely been used to separate glucose and fructose, and galactose and tagatose, as in US Patent, Nos., 3,416,961, 4,472,203, 8,802,843. Sodium has been used to separate glycerol and erythritol, as in US Patent No. 6,030,820.

In one embodiment of the current disclosure, a solution of L-threose and L-xylonic acid produced from the electrochemical decarboxylation of L-xylonic acid is eluted through a separation zone containing cation exchange resin. Water is the eluent and two product solutions are collected, one consisting primarily of L-threose and the other consisting primarily of acids. Preferably the cation exchange resin is in the same cationic form as the acids.

### Examples

### Example 1

A 2 Molar solution of xylonic acid was provided. This solution was used as the anolyte of a flow through electrochemical cell comprising a graphite foil anode, cation exchange membrane, and stainless steel cathode. The catholyte was a 3.5 molar solution of potassium hydroxide. The temperature of the anolyte was maintained at 31°C. A current of 90 mA/cm2 of geometric electrode surface was applied to the cell. Neutralization of the anolyte was maintained by addition of potassium hydroxide to the anolyte. The cell was run for 6.8 hrs. At the end of the reaction, there was 932 mmoles of L-xylonic acid, and 694 mmoles of L-threose.

### Example 2

A 2 Molar solution of potassium 2-keto-L-gulonic was provided with a pH of 6. This solution was used as the anolyte of a flow through electrochemical cell comprising a graphite foil anode, cation exchange membrane, and stainless steel cathode. The catholyte was a 3.5 molar solution of potassium hydroxide. The temperature of the anolyte was maintained at 45°C. A current of 100 mA/cm2 of geometric electrode surface was applied to the cell. Neutralization of the anolyte was maintained by addition of potassium hydroxide to the anolyte. The cell was run for 19.5 hrs. At the end of the reaction, there was 5.4 mmoles of KLG, 254 mmoles of L-xylonic acid, and 577 mmoles of L-threose.

### Example 3

350 ml of a 50 gram per liter solution of L-threose was provided from the electrochemical decarboxylation of KLG and added to a 1 L stirred tank pressure vessel. 2.8 g of a 4.5% gold catalyst, and 9.78 g of Magnesium Hydroxide were added to the solution. The vessel was purged once with oxygen and then pressurized to 100 psi with oxygen and stirred. The reaction was stopped after 3 hours. 98.6% of the L-threose was converted to magnesium L-threonate.

### Example 3

A 2 liter column was prepared and filled with cation resin Diaion UBK550 in the potassium form and maintained at 70°C. 150 ml of 25% brix solution of L-threose and L-xylonic acid was provided from an electrochemical decarboxylation and eluted with water at 0.54 bed volumes per hour. The % brix of the elution was measured using refractive index. The L-threose and L-xylonic acid were baseline resolved, and the L-threose fraction was collected from which 98% of the acids had been removed.

## Claims

1. A method of producing L-threonic acid from L-xylonic acid comprising:
providing L-xylonic acid,
decarboxylating L-xylonic acid to L-threose, and
oxidizing the L-threose to L-threonic acid.

2. The method of claim 1 wherein the L-xylonic acid is produced from the chemical decarboxylation of L-sorbose or KLG, wherein the term KLG refers to the 2-keto-L-gulonic acid, and salts thereof.

3. The method of claim 1 wherein the L-xylonic acid is produced from the oxidation of L-xylose.

4. The method of claim 1 wherein the L-xylonic acid is decarboxylated to threose using hypochlorous acid or hydrogen peroxide.

5. The method of claim 1 wherein the L-xylonic acid is decarboxylated to threose in an electrochemical cell.

6. The method of claim 5 wherein the L-xylonic acid is provided by the concurrent decarboxylation of KLG.

7. The method of claim 1 wherein the L-threose is oxidized to L-threonic acid using a catalyst in the presence of oxygen and a metal hydroxide.

8. The method of claim 2 wherein the L-threose is oxidized to L-threonic acid using a catalyst in the presence of oxygen and a metal hydroxide.

9. The method of claim 3 wherein the L-threose is oxidized to L-threonic acid using a catalyst in the presence of oxygen and a metal hydroxide.

10. The method of claim 4 wherein the L-threose is oxidized to L-threonic acid using a catalyst in the presence of oxygen and a metal hydroxide.

11. The method of claim 5 wherein the L-threose is oxidized to L-threonic acid using a catalyst in the presence of oxygen and a metal hydroxide.

12. The method of claim 6 wherein the L-threose is oxidized to L-threonic acid using a catalyst in the presence of oxygen and a metal hydroxide.

13. The method of any of claims 7 to 12 wherein the catalyst is gold-based.

14. The methods of any one of claims 1 to 13 wherein the threose is purified by eluting the decarboxylation solution through a separation zone containing cation exchange resin.

## Patentansprüche

1. Verfahren zum Herstellen von L-Threonsäure aus L-Xylonsäure umfassend:
Bereitstellen von L-Xylonsäure,
Decarboxylieren von L-Xylonsäure zu L-Threose, und
Oxidieren der L-Threose zu L-Threonsäure.

2. Verfahren nach Anspruch 1, wobei die L-Xylonsäure hergestellt wird aus der chemischen Decarboxylierung von L-Sorbose oder KLG, wobei der Ausdruck KLG die 2-Keto-L-gulonsäure betrifft, und Salzen davon.

3. Verfahren nach Anspruch 1, wobei die L-Xylonsäure aus der Oxidation von L-Xylose hergestellt wird.

4. Verfahren nach Anspruch 1, wobei die L-Xylonsäure zu Threose unter Verwendung von hypochloriger Säure oder Wasserstoffperoxid decarboxyliert wird.

5. Verfahren nach Anspruch 1, wobei die L-Xylonsäure zu Threose in einer elektrochemischen Zelle decarboxyliert wird.

6. Verfahren nach Anspruch 5, wobei die L-Xylonsäure durch die gleichzeitige Decarboxylierung von KLG bereitgestellt wird.

7. Verfahren nach Anspruch 1, wobei die L-Threose zu L-Threonsäure unter Verwendung eines Katalysators in der Gegenwart von Sauerstoff und eines Metallhydroxids oxidiert wird.

8. Verfahren nach Anspruch 2, wobei die L-Threose zu L-Threonsäure unter Verwendung eines Katalysators in der Gegenwart von Sauerstoff und eines Metallhydroxids oxidiert wird.

9. Verfahren nach Anspruch 3, wobei die L-Threose zu L-Threonsäure unter Verwendung eines Katalysators in der Gegenwart von Sauerstoff und eines Metallhydroxids oxidiert wird.

10. Verfahren nach Anspruch 4, wobei die L-Threose zu L-Threonsäure unter Verwendung eines Katalysators in der Gegenwart von Sauerstoff und eines Metallhydroxids oxidiert wird.

11. Verfahren nach Anspruch 5, wobei die L-Threose zu L-Threonsäure unter Verwendung eines Katalysators in der Gegenwart von Sauerstoff und eines Metallhydroxids oxidiert wird.

12. Verfahren nach Anspruch 6, wobei die L-Threose zu L-Threonsäure unter Verwendung eines Katalysators in der Gegenwart von Sauerstoff und eines Metallhydroxids oxidiert wird.

13. Verfahren nach einem der Ansprüche 7 bis 12, wobei der Katalysator auf Gold basiert.

14. Verfahren nach einem der Ansprüche 1 bis 13, wobei die Threose durch Eluieren der Decarboxylierungslösung durch eine Kationenaustauschharz enthaltende Trennzone gereinigt wird.

## Revendications

1. Procédé de production d'acide L-thréonique à partir d'acide L-xylonique comprenant :
la fourniture d'acide L-xylonique,
la décarboxylation de l'acide L-xylonique en L-thréose, et
l'oxydation du L-thréose en acide L-thréonique.

2. Procédé selon la revendication 1, dans lequel l'acide L-xylonique est produit par la décarboxylation chimique du L-sorbose ou de KLG, où le terme KLG désigne l'acide 2-céto-L-gulonique, et ses sels.

3. Procédé selon la revendication 1, dans lequel l'acide L-xylonique est produit par l'oxydation du L-xylose.

4. Procédé selon la revendication 1, dans lequel l'acide L-xylonique est décarboxylé en thréose à l'aide d'acide hypochloreux ou de peroxyde d'hydrogène.

5. Procédé selon la revendication 1, dans lequel l'acide L-xylonique est décarboxylé en thréose dans une cellule électrochimique.

6. Procédé selon la revendication 5, dans lequel l'acide L-xylonique est fourni par la décarboxylation simultanée de KLG.

7. Procédé selon la revendication 1, dans lequel le L-thréose est oxydé en acide L-thréonique à l'aide d'un catalyseur en présence d'oxygène et d'un hydroxyde métallique.

8. Procédé selon la revendication 2, dans lequel le L-thréose est oxydé en acide L-thréonique à l'aide d'un catalyseur en présence d'oxygène et d'un hydroxyde métallique.

9. Procédé selon la revendication 3, dans lequel le L-thréose est oxydé en acide L-thréonique à l'aide d'un catalyseur en présence d'oxygène et d'un hydroxyde métallique.

10. Procédé selon la revendication 4, dans lequel le L-thréose est oxydé en acide L-thréonique à l'aide d'un catalyseur en présence d'oxygène et d'un hydroxyde métallique.

11. Procédé selon la revendication 5, dans lequel le L-thréose est oxydé en acide L-thréonique à l'aide d'un catalyseur en présence d'oxygène et d'un hydroxyde métallique.

12. Procédé selon la revendication 6, dans lequel le L-thréose est oxydé en acide L-thréonique à l'aide d'un catalyseur en présence d'oxygène et d'un hydroxyde métallique.

13. Procédé selon l'une quelconque des revendications 7 à 12, dans lequel le catalyseur est à base d'or.

14. Procédés selon l'une quelconque des revendications 1 à 13, dans lesquels le thréose est purifié par élution de la solution de décarboxylation à travers une zone de séparation contenant une résine échangeuse de cations.
